# EUROPÄISCHE PATENTSCHRIFT

(11) **EP 2 825 265 B1**
(45) Veröffentlichungstag und Bekanntmachung des Hinweises auf die Patenterteilung: **01.11.2017**
(21) Anmeldenummer: 13711003.7
(22) Anmeldetag: 15.03.2013
(51) Int. Cl.: A61Q 19/00, A61K 8/97, A61Q 19/08, A61P 17/16, A61K 36/73, A61K 8/63, A61K 8/67, A61K 8/92

(54) **KOSMETIKPRODUKTE FÜR DIE ALTERSHAUT**
COSMETIC PRODUCTS FOR SKIN AGEING
PRODUIT COSMÉTIQUE POUR PEAU MÂTURE

(30) Priorität: 15.03.2012 EP 12159563
(43) Veröffentlichungstag der Anmeldung: 21.01.2015
(73) Patentinhaber: Kneipp GmbH, 97084 Würzburg (DE)
(72) Erfinder: WOHLFART, Rainer, 97320 Sulzfeld am Main (DE); BLAAK, Jürgen, 97084 Würzburg (DE); SIMON, Isabel, 97252 Frickenhausen am Main (DE); STAIB, Peter, 97320 Sulzfeld am Main (DE)
(74) Vertreter: Keller, Günter
(86) Internationale Anmeldenummer: PCT/EP2013/055316
(87) Internationale Veröffentlichungsnummer: WO 2013/135854

(56) Entgegenhaltungen:
- DE-A1-102006 051 685
- DE-C1- 19 712 659
- FR-A1- 2 903 902
- FR-A2- 2 294 714
- US-A- 4 054 649
- US-B1- 6 572 868
- CHOI EUNG-HO ET AL: "Stratum corneum acidification is impaired in moderately aged human and murine skin", JOURNAL OF INVESTIGATIVE DERMATOLOGY, NATURE PUBLISHING GROUP, GB, Bd. 127, Nr. 12, 1. Dezember 2007 (2007-12-01), Seiten 2847-2856, XP002522058, ISSN: 0022-202X, DOI: 10.1038/SJ.JID.5700913 [gefunden am 2007-06-07] in der Anmeldung erwähnt
- ROSHAN GUNATHILAKE ET AL: "pH-Regulated Mechanisms Account for Pigment-Type Differences in Epidermal Barrier Function", JOURNAL OF INVESTIGATIVE DERMATOLOGY, Bd. 129, Nr. 7, 29. Januar 2009 (2009-01-29), Seiten 1719-1729, XP055032592, ISSN: 0022-202X, DOI: 10.1038/jid.2008.442 in der Anmeldung erwähnt
- Stephanie Ivancheva ET AL: "Pharmacological activities and biologically active compounds of Bulgarian medicinal plants", Phytochemistry: Advances in Research, 1. Januar 2006 (2006-01-01), Seiten 87-103, XP055032579, ISBN: 978-8-13-080034-9 Gefunden im Internet: URL:http://www.trnres.com/ebook/uploads/im perato/T_1231133680Imperato-4.pdf [gefunden am 2012-07-12]

## Beschreibung

Das Durchschnittsalter der Bevölkerung steigt in den Industrieländern permanent an. Insbesondere die Zahl der über 60-jährigen wird in den nächsten Jahrzehnten stark zunehmen. Für diese Bevölkerungskreise müssen Produkte bereitgestellt werden, die den speziellen Anforderungen gerecht werden. Gerade bei alten und insbesondere bei sehr alten Menschen ist häufig zu beobachten, dass die Haut geschädigt ist oder durch leichteste Beanspruchungen beschädigt wird. Bei der Altershaut ist der physiologische pH-Wert häufig erhöht (Choi et al. (2007): Stratum corneum acidification is impaired in moderately aged human and murine skin. J Invest Dermatol 127: 2847-2856).

Aus dem Stand der Technik sind auch verschiedenste kosmetische Zusammensetzungen bekannt, die unterschiedliche Komponenten und Zusatzstoffe enthalten.

Die FR 2903902 beschreibt die Verwendung von einer kosmetischen Zusammensetzung zur Depigmentierung der Haut, wobei ein wässriger Extrakt von Frauenmantel eingesetzt wird.

Die US 6,572,862 B1 offenbart Zusammensetzungen, die die Haut vor dem Altern schützen sollen. Neben vielen unterschiedlichen Pflanzenextrakten werden auch Extrakte von Frauenmantel erwähnt.

Die US 4,054,649 B1 offenbart die Verwendung von Alchemilla bei der Behandlung von Bindegewebeverletzungen, die beispielsweise im Zusammenhang mit Schwangerschaften auftreten.

Die FR 2294714 offenbart die Verwendung von alkoholischem Frauenmantelextrakt.

Die DE 197 12 659 C1 beansprucht die Verwendung eines Extraktes von Alchemilla vulgaris zur Hemmung der Angiogenese.

Choi et al., J. Invest. Dermatol.(2007),Vol. 127,S.2847-2856 weisen darauf hin, dass die Barriereeigenschaften bei der älteren Haut gestört sein können.

Guanthilake et al., J Invest. Dermatol.(2009),Vol. 129(7):1719-1729 untersuchen die Barrierefunktion der Haut in Abhängigkeit von den Pigmentierungstypen. Stärker pigmentierte Probanden zeigten eine schnellere Regeneration der Barrierefunktion der Haut.

Gegenstand der vorliegenden Erfindung sind Kosmetikprodukte gemäß den Patentansprüchen mit einem niedrigen pH-Wert, die einen positiven Einfluss auf epidermale Strukturen der Altershaut haben. Diese Produkte schützen, stabilisieren und erhöhen die Funktion der epidermalen

Permeabilitätsbarriere. Bevorzugt handelt es sich bei den Kosmetikprodukten um barrierestabilisierende Emulsionen.

Der saure Hautoberflächen pH-Wert ist seit langem bekannt und liegt neuesten Studien zufolge zwischen 4,5 und 5. Dieser saure pH-Wert der Hautoberfläche steigt von außen nach innen an und beträgt zwischen Stratum corneum (Hornzellschicht) und Stratum granulosum (Körnerzellschicht) etwa 7, Durch diesen pH-Gradienten werden unterschiedliche Enzyme mit unterschiedlichem pH-Optimum gezielt in ihrer Aktivität gesteuert.

Enzyme mit einem pH-Optimum zwischen 4 und 5, wie z.B. β-Glucocerebrosidase und saure Sphingomyelinase sind von entscheidender Bedeutung für die Formation der interzellulären Lipidmatrix der epidermalen Permeabilitätsbarriere, die im Stratum corneum (SC) lokalisiert ist.

Ferner sind an der Desquamation (Abschuppung) der Hornzellen Serinproteasen beteiligt, die ein pH-Optimum von 7 haben. Bei einem Hautoberflächen-pH von 4,5 bis 5 sind diese Enzyme in ihrer Aktivität gehemmt, so dass es nicht zu einer übermäßigen Desquamation und damit zu einem Ungleichgewicht zwischen Desquamation und Zellerneuerung kommt.

Darüber hinaus wirkt der saure Hautoberflächen-pH antimikrobiell, Die Normalflora der Haut hat sich dem pH 5 angepasst. Im Gegensatz dazu ist das Wachstum pathogener Keime, wie z.B. Staphylococcus aureus, bei pH 5 gehemmt. Somit sorgt der Hautoberflächen-pH zwischen 4,5 und 5 für eine Stabilisierung der "nützlichen" Normalflora und für eine Abwehr pathogener Keime. Ferner ist nachgewiesen, dass eine Erhöhung des Hautoberflächen-pH die unerwünschte Dissoziation (Entfernung) der Normalflora begünstigt.

Zusammenfassend ist der Hautoberflächen-pH zwischen 4,5 und 5 von zentraler Bedeutung für die Funktion der epidermalen Permeabilitätsbarriere, denn er reguliert die Barriereintegrität (Widerstandskraft der Barriere), die Barrierekohäsion (Zusammenhalt der Barriere), die Barriereregeneration nach Schädigung, sowie die antimikrobielle Abwehr.
(1) Eine Erhöhung des Hautoberflächen-pH hemmt die β-Glucocerebrosidase und die saure Sphingomyelinase und damit die Formation der interzellulären Lipidmatrix, der epidermalen Permeabilitätsbarriere.
(2) Eine Erhöhung des Hautoberflächen-pH steigert die Aktivität der Serinproteasen, gefolgt von einer übermäßigen Desquamation und damit verminderten Barriereintergrität und -kohäsion.
(3) Die Erhöhung des Hautoberflächen-pH hemmt das Wachstum der Normalflora, begünstigt das Wachstum pathogener Keime und dadurch bedingt die Entstehung dermaler Infektionen.

Bei den entzündlichen Hauterkrankungen (z.B. Ekzemerkrankungen oder Akne) ist der saure Hautoberflächen-pH erhöht. Bei der Haut von Neugeborenen (< 6 Wochen) und der Altershaut (>70 Jahre) liegt der Hautoberflächen-pH bei etwa 6-7.

In den 60er Jahren kamen zunehmend Hautreinigungsprodukte mit definiertem pH-Wert auf den Markt. Hintergrund war die Erkenntnis, dass der Hautoberflächen-pH-Wert stark durch Externa beeinflusst werden kann. Es wurden "pH-hautneutrale" Produkte zur Hautreinigung entwickelt. Diese Produkte waren und sind für die normale Haut bestimmt und sollen den normalen Hautoberflächen-pH stabilisieren. Von einigen Herstellern werden diese Produkte auch für empfindliche oder trockene Haut empfohlen.

Ein weiteres Gebiet, auf dem Kosmetika mit definiertem pH-Wert vermarktet werden, ist der Bereich des "Chemical Peeling". Durch das "Chemical Peeling" wird eine Ablösung der Hornzellen erreicht (Keratolyse), wodurch eine epidermale und dermale Regeneration angeregt wird. Das "Peeling" selbst wird mit extrem sauren Lösungen (pH-Wert 1-2) in der Arztpraxis durchgeführt. Die Vor-, Zwischen- und Nachversorgung der Patienten erfolgt mit Kosmetikprodukten mit einem pH-Wert von 3 bis 4.

Die erfindungsgemäßen Kosmetikprodukte unterscheiden sich von beiden oben beschriebenen Ausführungsformen:
1. Die erfindungsgemäßen Kosmetikprodukte sind nicht primär für die Anwendung an normaler Haut bestimmt, sondern eine spezielle Entwicklung für spezielle Hautzustände und inflammatorische Hauterkrankungen, die insbesondere bei einer reduzierten epidermalen Barrierefunktion auftreten.
2. Die Erfindung ist vom "Chemical Peeling" abzugrenzen, denn es soll durch die Erfindung gerade keine Keratolyse erreicht werden, daher haben die Produkte einen pH-Wert von wenigstens 3,2 und besonders bevorzugt von wenigstens 3,5.
3. Die erfindungsgemäßen Produkte dienen einer speziellen Hautpflege für Hautzustände, deren gemeinsames Merkmal ein erhöhter Hautoberflächen-pH ist. Darüber hinaus ist den oben beschriebenen Hautzuständen gemeinsam, dass eine verminderte antimikrobielle Barriere und somit eine gesteigerte Anfälligkeit für Infektionen vorliegt.

Durch die Applikation der erfindungsgemäßen Kosmetikprodukte, insbesondere Cremes, Lotionen, Waschlotionen mit einem niedrigen pH-Wert kann der erhöhte Hautoberflächen-pH bestimmter Hautzustände und Hauterkrankungen in den normalen (physiologischen) Bereich zwischen 4,5 und 5 verschoben, respektive normalisiert und stabilisiert werden.

Durch die Normalisierung des Hautoberflächen-pH kann insbesondere die Funktion der epidermalen Permeabilitätsbarriere (Barriereintegrität, -kohäsion und -regeneration, relative Hornschichtfeuchte) verbessert werden, und die Funktion der antimikrobiellen Barriere verbessert werden.

Erfindungsgemäß wurden allgemein anerkannte Messverfahren der dermatologischen Forschung eingesetzt. Es wurden der Hautoberflächen-pH (Skin pH-Meter® 905; Courage & Khazaka, Köln, Deutschland), die relative Hornschichtfeuchte (Corneometer® CM 825; Courage & Khazaka, Köln, Deutschland), sowie der transepidermale Wasserverlust (Tewameter® TM 300; Courage & Khazaka, Köln, Deutschland) nach entsprechenden Richtlinien ermittelt (Parra et al. (2003): EEMCO guidance for the in vivo assessment of skin surface pH. Skin Pharmacol Appl Skin Physiol 16: 188-202, Berardesca (1997): EEMCO guidance for the assessent of stratum corneum hydration: electrical methods. Skin Res Technol 3: 126-132, Rogiers (2001): EEMCO guidance for the assessment of transepidermal water loss in cosmetic sciences. Skin Pharmacol Appl Physiol 14: 117-128). Darüber hinaus wurde die Überprüfung der epidermalen Barriereintegrität in Anlehnung an bereits publizierte Studien durchgeführt (Gunathilake et al. (2009): pH-Regulated Mechanisms Account for Pigment-Type Differences in Epidermal Barrier Function. J Invest Dermatol 129(7): 1719-1729).

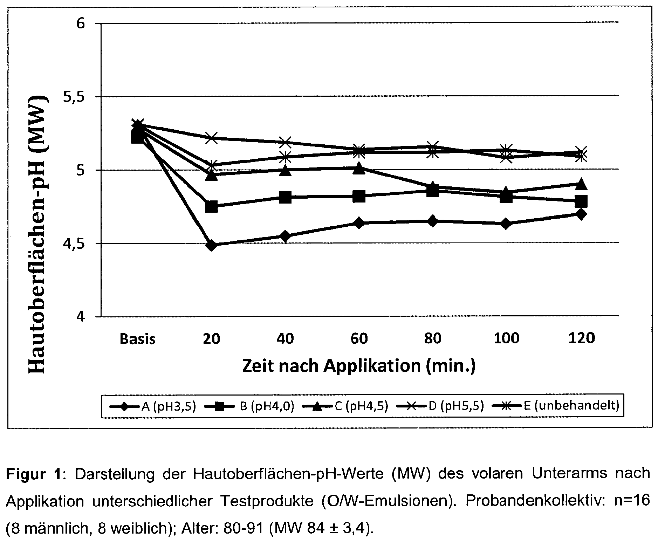

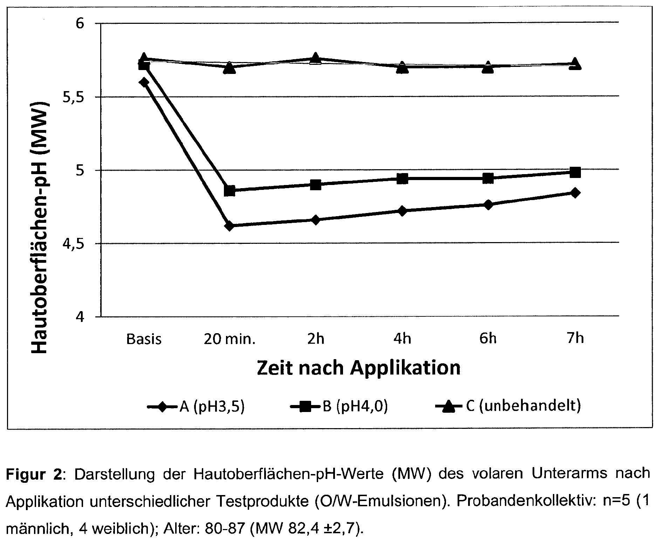

Die Überprüfung der kosmetischen Produkte wurde exemplarisch an der Haut von über 80-jährigen (Hochbetagten) durchgeführt. In Untersuchungen an der Haut von über 80-jährigen wurde gezeigt, dass mit einem erfindungsgemäßen kosmetischen Produkt der erhöhte Hautoberflächen-pH der Altershaut in den physiologischen Bereich zwischen pH 4,5 und 5 verschoben werden kann. Nach einmaligem Auftragen wurde der altersbedingt erhöhte Hautoberflächen-pH über 2 (Figur 1) bzw. über 7 Stunden (Figur 2) erniedrigt. Somit reicht wahrscheinlich die zweimalige tägliche Applikation des Pflegeproduktes aus, um eine kontinuierliche Normalisierung bzw. Stabilisierung des Hautoberflächen-pH zu erreichen. Dieser Effekt wurde durch eine handelsübliche Creme mit pH 5,5 nicht erreicht (Figur 1). Ferner wurde durch die 4-wöchige zweimalige tägliche Anwendung eines kosmetischen Produkts mit pH4 die Funktion der epidermalen Permeabilitätsbarriere verbessert. Es wurde untersucht, ob die 4-wöchige Anwendung des kosmetischen Produkts mit pH4 die Barriereintegrität der Altershaut verbessert.

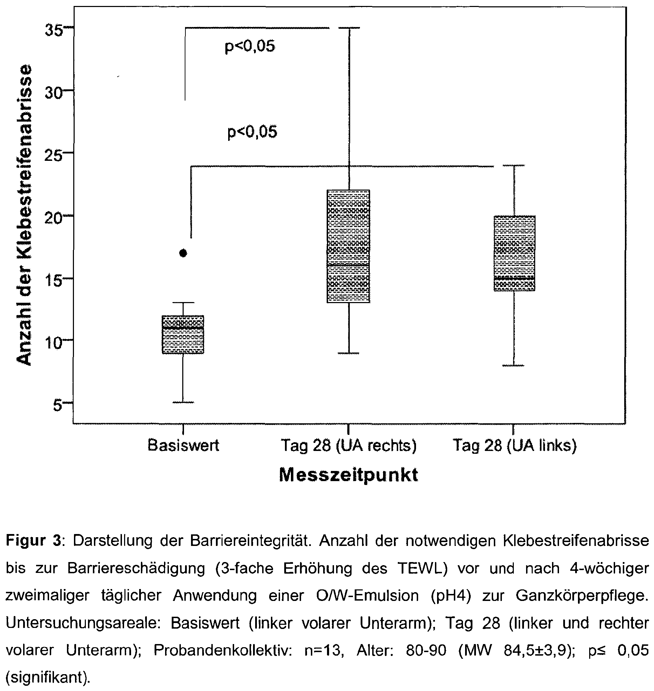

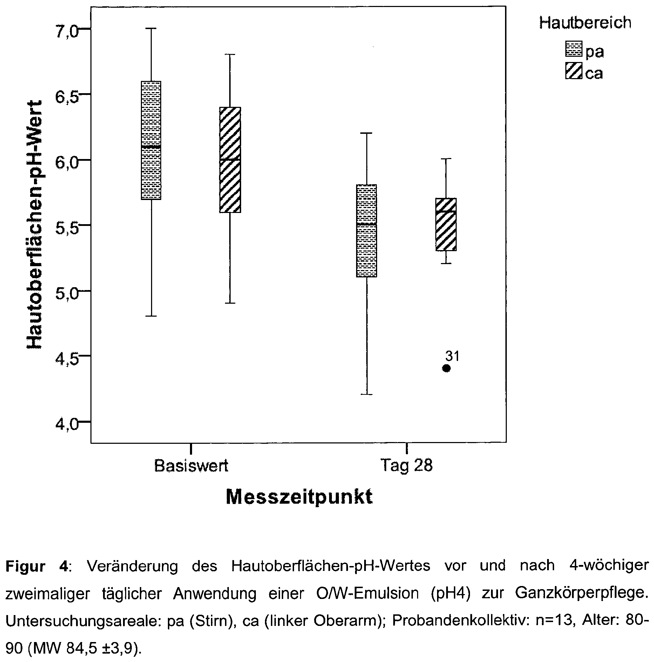

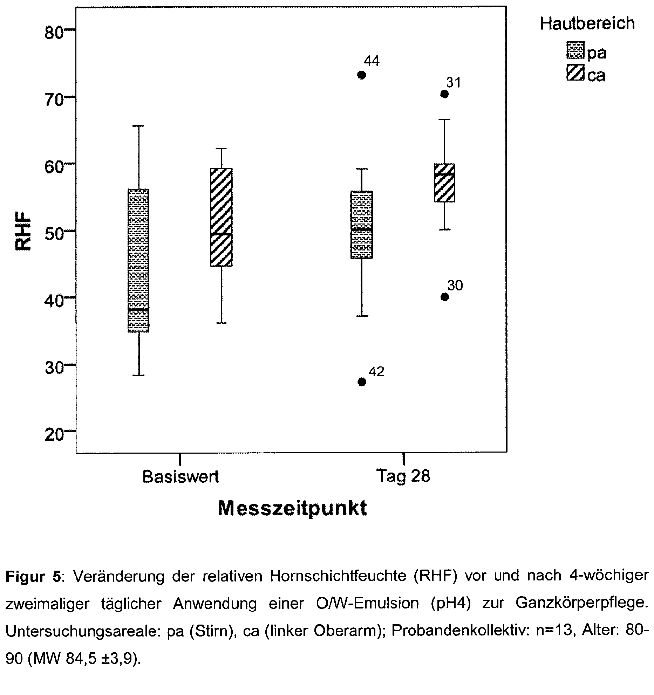

Die Barriereintegrität wurde überprüft, indem am Unterarm mit einem Klebestreifen (3M Blenderm surgical tape; 3M Deutschland; Neuss; Deutschland) Abrisse vorgenommen wurden. Das Abrissprocedere erfolgte in Anlehnung an Dickel et al. (2004): The "strip" patch test: results of a multicentre study towards a standardization. Arch Dermatol Res 296(5): 212-219, bis der transepidermale Wasserverlust (TEWL) um das 3-fache erhöht war. Die 3-fache Erhöhung des TEWL gilt als Indiz für eine epidermale Barriereschädigung (Gunathilake et al. (2009): pH-Regulated Mechanisms Account for Pigment-Type Differences in Epidermal Barrier Function. J Invest Dermatol 129(7): 1719-1729). Je weniger Abrisse erfolgen müssen um die Barriere zu schädigen (3-fache TEWL-Erhöhung), desto schlechter ist die Barriereintegrität. Vor der 4-wöchigen Therapie (s.o.) wurden durchschnittlich (Medianwert) 11 Klebestreifenabrisse benötigt um die Barriere zu schädigen, nach der Therapie waren 16 (rechter Unterarm) bzw. 15 (linker Unterarm) Klebestreifenabrisse notwendig (Figur 3). Das bedeutet, dass durch die 4-wöchige Anwendung eines kosmetischen Produkts mit pH4 bei über 80-jährigen (n=13) die Barriereintegrität signifikant verbessert wurde. Die 4-wöchige Anwendung des kosmetischen Produkts mit pH4 führte ferner zu einer signifikanten Erniedrigung des Hautoberflächen-pH und zu einer signifikanten Erhöhung der relativen Hornschichtfeuchte (Figuren 4-6).

Durch die erfindungsgemäßen Produkte wird die Beeinflussung und Stabilisierung des Hautoberflächen-pH von 4,5-5,0 durch die externe Zuführung von H⁺-Ionen erreicht, die den Produkten zugegeben werden. In bevorzugter Weise wird die Absenkung der pH-Werte durch Zugabe von physiologisch unbedenklichen Säuren, wie Milchsäure, Zitronensäure, Apfelsäure, Weinsäure, Salzsäure oder Phosphorsäure oder deren Salze, wie beispielsweise Di-Natriumhydrogenphosphat oder Natriumdihydrogenphosphat bewirkt.

Bei den Versuchen zum Einfluss des pH-Wertes wurden kosmetische Produkte verwendet, die hinsichtlich sämtlicher Komponenten identisch waren und sich nur im pH-Wert unterschieden. Die Einstellung des pH-Wertes erfolgte mit Zitronensäure. Der pH-Wert wurde nach auf dem Gebiet üblichen Methoden bestimmt und die Stabilität des eingestellten pH-Wertes wurde überwacht.

Gegenstand der vorliegenden Erfindung sind kosmetische Produkte, bevorzugt barrierestabilisierende Emulsionen, die als Wirkstoff einen Extrakt aus Frauenmantel (Alchemilla vulgaris) mit standardisiertem Polyphenolgehalt, bevorzugt in einer Menge von 0,025 bis 3 Gew.-%, besonders bevorzugt von 0,05 bis 0,5 Gew.-% und ganz besonders bevorzugt von 0,075 bis 0,2 Gew.-% des Trockenextrakts bezogen auf das fertige Produkt enthalten.

Bei den erfindungsgemäßen kosmetischen Produkten handelt es sich in bevorzugter Ausführungsform um barrierestabilisierende Emulsionen, wobei es sich hierbei in bevorzugter Ausführungsform um Emulsionen, in Form von Lotionen oder Cremes handelt.

Im Rahmen der vorliegenden Erfindung wurden verschiedene, auf unterschiedliche Art und Weise hergestellte Extrakte der Pflanze Alchemilla vulgaris hinsichtlich Phototoxizität, Zytotoxizität und antioxidativer Kapazität getestet. Bei diesen Versuchen stellte sich heraus, dass in besonders bevorzugter Ausführungsform ein wässriger Trockenextrakt aus Alchemilla vulgaris eine ausgeprägte antioxidative Kapazität aufweist.

Als Maß für die antioxidative Kapazität des erfindungsgemäß bevorzugt eingesetzten Trockenextrakts wurde der sogenannte ORAC-Wert (Oxygen Radical Absorbance Capacity) untersucht. Hierbei stellte sich heraus, dass der ORAC-Wert des erfindungsgemäß eingesetzten Trockenextrakts bevorzugt wenigstens 100000, besonders bevorzugt wenigstens 200000 und ganz besonders bevorzugt wenigstens 250000 µmol Trolox Äquivalente (TE)/100 g Extrakt aufweist. Die erfindungsgemäß eingesetzten Extrakte weisen einen nach der Methode ORAC FL gemessenen Wert von wenigstens 250000 µmol TE / 100 g, bevorzugt von wenigstens 280000 µmol TE / 100 g auf. Die Ergebnisse sind angegeben als "Trolox Äquivalente" (TE). Bei der Testmethode wird der oxidative Abbau eines fluoreszierenden Moleküls (beispielsweise Fluorescein) nach Mischung mit einer freie Radikale erzeugenden Verbindung gemessen.

Eine weitere, besonders bevorzugte Wirkkomponente, die den erfindungsgemäßen kosmetischen Produkten zugesetzt wird, ist Vitamin E sowie kosmetisch akzeptable Derivate hiervon. Ein besonders bevorzugtes kosmetisch annehmbares Derivat ist das Tocopherylacetat, das auch als Vitamin E-Acetat bezeichnet wird. Ein Vorteil des erfindungsgemäß eingesetzten Tocopherylacetats ist, dass es in den kosmetischen Produkten nicht oxidiert wird und die äußeren Schichten der Epidermis durchdringen kann. Erfindungsgemäß eingesetzt wird Vitamin E bzw. Tocopherylacetat in einer Menge von 0,01-5 Gew.-%, bevorzugt 0,2-3,0 Gew.-% und besonders bevorzugt 1,5-2,5 Gew.-%. Die Gewichtsprozentangaben beziehen sich jeweils auf das fertige Produkt.

In bevorzugter Ausführungsform weisen die erfindungsgemäßen barrierestabilisierende Emulsionen die im Folgenden aufgeführten Komponenten in den jeweiligen Anteilen bezogen auf das fertige Produkt auf:
- Emulgatoren werden in einer Menge von 1,5 bis 8, bevorzugt 2,5 bis 7 und besonders bevorzugt 3,0 bis 6,0 Gew.-% eingesetzt. Bei den Emulgatoren handelt es sich teilweise auch um Mischungen der im Folgenden aufgeführten Komponenten: Polyoxyethylenfettalkoholether, Polyglykolfettsäureester, Polyoxyethylenester mit modifizierten Fettsäuren; Cholesterol und Fettsäureester, Glyceryldilaurat, Glykolstearat, Dinatriumlaurethsulfosuccinat, Natriumdioctylsulfosuccinat, Alkoholethersulfat, Natriumalkylarylsulfonat, Polyethylenglykolalkylamine, quaternäre Ammoniumsalze, Cetearylalkohol und Polysorbat 20, Glycerylstearat, Kaliumpalmitoyl; hydrolysiertes Weizenprotein, hydrogeniertes Lecithin, Glyceryloleate.
- Mischungen verschiedener ungesättigter oder mehrfach ungesättigter Fettsäuren werden in einer Menge von 1,0 bis 40, bevorzugt 10 bis 35 und besonders bevorzugt 15 bis 25 Gew.-% eingesetzt. Die Fettsäuren können in der Form von Fettsäuren, Fettsäuresalzen oder in Form der Ester mit mehrwertigen Alkoholen wie Glycerin eingesetzt werden.
- Als Verdicker werden 0,1 bis 2, bevorzugt 0,2 bis 1 Gew.-% und besonders bevorzugt 0,25 bis 0,45 Gew.-% der folgenden Komponenten entweder einzeln oder im Gemisch eingesetzt: mikrokristalline Zellulose, Algin, Xanthangummi oder kosmetisch akzeptable Acrylate, wie C₁₀-C₃₀-Alkylacrylat-Crosspolymere.
   Als Feuchthaltemittel wird in bevorzugter Ausführungsform 1,0 bis 20, bevorzugt 3,0 bis 10 Gew.-% und besonders bevorzugt 5,0 bis 8,0 Gew.-% wässriges Glycerin (85 % Glycerin) in pharmazeutisch anwendbarer Qualität zugegeben.
- Wenn zusätzlich noch Gerbstoffe eingesetzt werden, werden diese in einer Menge von 0,01 bis 1, bevorzugt 0,05 bis 0,5 und besonders bevorzugt 0,1 bis 0,2 Gew.-% zugegeben. Bevorzugte Polyphenole, wie Gerbstoffe sind charakteristisch für den Frauenmantel (*Alchimella vulgaris*)*.* Frauenmantel zeichnet sich aus durch einen hohen Gehalt an Polyphenolen aus der Gruppe der Ellagtannine und Gallotannine (insgesamt zwischen 6-8%) aus. Wenn man den Gehalt der einzelnen Komponenten in Gewichtsprozent bezogen auf Drogentrockenextrakt angibt, weist der FrauenmantelTrockenextrakt, der bevorzugt erfindungsgemäß eingesetzt wird, einen Gehalt von 6-8 Gew.-% an Gerbstoffen auf. Bestandteile der Gerbstoffe sind Gallotannine, die in einer Menge von 1,0 bis zu 3,0 Gew.-%, bezogen auf den Drogentrockenextrakt vorliegen, sowie höhere Mengen an Ellagtanninen. Bei den Ellagtanninen handelt es sich insbesondere um Pedunculagin, das in einer Menge von 0,8 bis 1,5 Gew.-%, bevorzugt 1,0 bis 1,2 Gew.-%, bezogen auf den Drogentrockenextrakt vorliegt, um Agrimoniin, das in einer Menge zwischen 1,0 und 4,0 Gew.-%, bevorzugt 2,3 bis 3,6 Gew.-%, bezogen auf den Drogentrockenextrakt vorliegt sowie Laevigatin, das in einer Menge von 0,8 bis 1,0 Gew.-%, bevorzugt 0,85 bis 0,95 Gew.-% vorliegt.

Eine besonders bevorzugte barrierestabilisierende Emulsion der vorliegenden Erfindung beinhaltet folgende Komponenten in den angegebenen Mengenbereichen:

| **Bestandteil** | **Anteil in Gew.-%** |
|---|---|
| Emulgator | 4 |
| Pflanzenöl | 20 |
| Alchemilla vulgaris Trockenextrakt | 1% |
| Zitronensäure | zur pH Einstellung |
| pH-Wert | 4,0 ± 0,2 |

Die Ergebnisse der Beispiele sind in den Figuren dargestellt.
Figur 1 gibt den Hautoberflächen-pH des volaren Unterarms nach Applikation verschiedener Testprodukte über einem Zeitraum von 2 Stunden wieder. Es handelte sich hierbei um Öl in Wasseremulsionen, die sich lediglich im pH-Wert unterschieden. Weitere Wirkstoffe wurden hier nicht zugesetzt.
Figur 2 stellt die Auswirkung einer Creme mit niedrigem pH über einige Zeit nach der Applikation (bis zu sieben Stunden) dar. Aus Figur 2 ist ersichtlich, dass durch Verabreichung eines erfindungsgemäßen kosmetischen Produkts eine Absenkung des Hautoberflächen-pH unter 5 für mehrere Stunden bewirkt werden kann.
Figur 3 zeigt, dass nach 4-wöchiger Behandlung mit einem Produkt mit einem pH-Wert von 4 die epidermale Barriereintegrität verbessert werden konnte. Der Basiswert stellt die Kontrolle, also unbehandelte Haut dar.
Figur 4 zeigt, dass nach 4-wöchiger Behandlungsdauer bei zweimal täglicher Applikation der Hautoberflächen-pH abgesenkt werden konnte. Gemessen wurde der pH-Wert an überwiegend lichtgealterter (Stirn, zentral) und vergleichsweise an überwiegend zeitgealterter (Oberarm, ventral) Haut.
Figur 5 zeigt den positiven Effekt einer Creme mit pH 4 bei zweimaliger täglicher Applikation über eine Dauer von vier Wochen im Hinblick auf die Erhöhung der relativen Hornschichtfeuchte. Auch hier wurde an zwei verschiedenen Körperstellen (Stirn, Oberarm) gemessen.

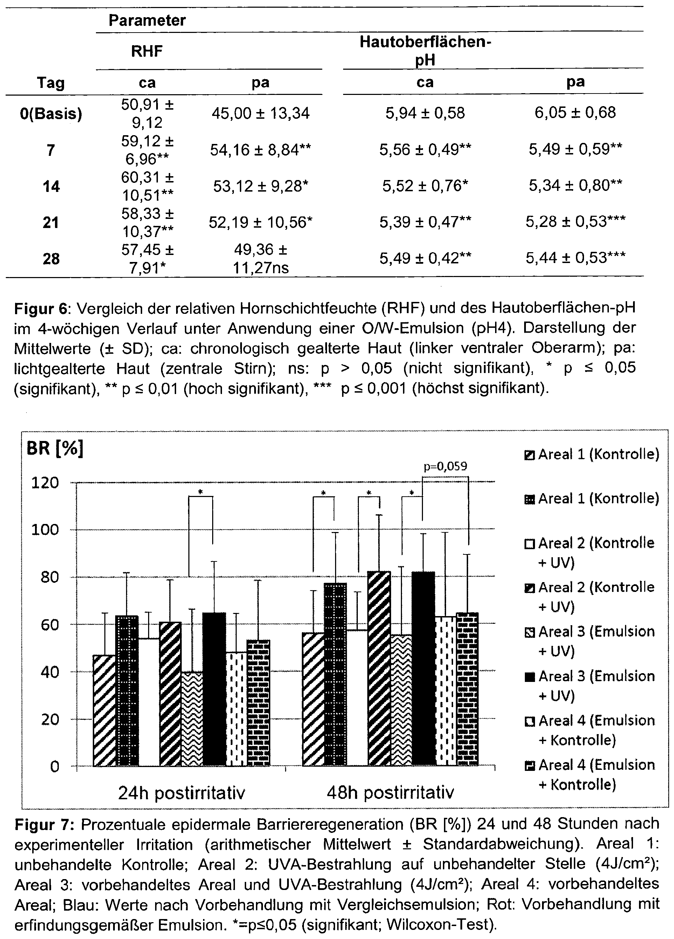

Bei Figur 6 sind die relativen Hornschichtfeuchtwerte und Hautoberflächen-pH unterschiedlicher Messzeitpunkte in tabellarischer Form dargestellt.

Im Rahmen der vorliegenden Erfindung konnte eine deutliche Verbesserung der Wirksamkeit durch den Zusatz eines Extraktes aus Frauenmantel erzielt werden.

Gegenstand der vorliegenden Erfindung sind daher kosmetische Produkte für die Altershaut mit einem pH-Wert von 3,0-5,2, die 0,1-5 Gew.-% eines Extraktes aus Frauenmantel bezogen auf das fertige Produkt enthalten.

Die erfindungsgemäßen Produkte weisen einen Anteil von 0,1-5 Gew.-%, bevorzugt 0,2-2,5 Gew.-% und besonders bevorzugt 0,2-1,0 Gew.-% eines Extraktes aus Frauenmantel auf, bezogen auf das fertige Produkt. Dies bedeutet, dass ein erfindungsgemäßes kosmetisches Produkt 0,1-5 g Extrakt aus Frauenmantel bezogen auf 100 g fertiges kosmetisches Produkt beinhaltet.

Frauenmantel gehört zu den Rosaceae und beinhaltet pharmakologisch aktive Inhaltsstoffe, insbesondere Gerbstoffe wie Gallotannine, Ellagitannine (Agrimoniin, Laevigatin, Pedunculagin), Flavonoide (Quercetinglykoside, Leukocyanidin), Phytosterine, sowie ätherische Öle. Diese Stoffe werden bei dem erfindungsgemäß eingesetzten Trockenextrakt angereichert.

Im Rahmen der vorliegenden Erfindung wird ein Trockenextrakt aus Frauenmantelextrakt eingesetzt, weil dieser eine besonders gute Wirksamkeit hinsichtlich der antioxidativen Kapazität und der antizytotoxischen Wirksamkeit hat. Dies führt zu einer überraschend deutlichen Erhöhung der protektiven Wirksamkeit. Eine antioxidative Wirkung eines Extraktes von Alchemilla xantochlora wird von Filipek J et al., Pharmazie 47: 717-718 (1992): The effect of Alchimella xantochlora water extract on lipid peroxidation and superoxide anion scavenging activity. Pharmazie 47: 717-718) beschrieben.

Im Rahmen der vorliegenden Erfindung wurde überraschenderweise festgestellt, dass in kosmetischen Produkten, die auf einen pH-Wert zwischen 3,2 und 4,8 eingestellt wurden, durch den Zusatz eines wässrigen Extraktes von Frauenmantel eine wesentliche barrierestabilisierende Wirkung erzielt werden kann. Dieser Effekt kann durch die Zugabe von Vitamin E bzw. Tocopherolacetat weiter verbessert werden. Diese Ergebnisse treten zwar auch bei normaler und gesunder Haut auf, sind aber besonders ausgeprägt bei der Altershaut und ganz besonders ausgeprägt bei der geschädigten Altershaut. Bei Schädigungen der Altershaut treten vor allem Probleme auf, wie Xerosis cutis, Pruritus, Kontaktdermatitis, Ekzeme oder Pilzbefall, wie Candiasis.

In bevorzugter Ausführungsform wird der Trockenextrakt durch eine wässrige Extraktion der Droge erhalten. Hierbei werden die Pflanze bzw. die pharmazeutisch verwendbaren Pflanzenteile gesammelt und getrocknet. Die getrockneten und zerkleinerten Pflanzenteile werden dann mit Wasser extrahiert, wobei eine leichte Erwärmung bevorzugt auf eine Temperatur zwischen 30 und 50°C für die Effizienz der Extraktion hilfreich ist.

Die Wirkstoffe können aus der getrockneten oder auch frischen Pflanze nach verschiedenen an sich bekannten Verfahren hergestellt werden. Bevorzugt eingesetzt wird erfindungsgemäß ein wässriger Extrakt, bei dem die schonend getrockneten Pflanzenbestandteile, insbesondere die während der Blütezeit gesammelten oberirdischen Pflanzenteile mit Wasser extrahiert werden. Hierzu werden die Pflanzenteile schonend, gegebenenfalls unter Stickstoffatmosphäre zerkleinert und mit Wasser extrahiert. Die Pflanzenteile werden einige Zeit (1-12 Stunden) in Wasser belassen, wobei eine milde Erwärmung auf bevorzugt 30°C bis maximal 50°C bevorzugt wird. Anschließend wird das Wasser von den wasserlöslichen Bestandteilen getrennt und die wässrige Phase zur Trockene eingeengt. Wenn es sich in den Herstellungsprozess gut einfügt, kann auch wässriger Extrakt direkt in die kosmetischen Produkte eingearbeitet werden. Wichtig ist aber, dass die flüssigen Extrakte hinsichtlich der Trockenbestandteile standardisiert werden, so dass die erfindungsgemäße Konzentration an Frauenmantelextrakt eingehalten werden kann.

In bevorzugter Ausführungsform weisen die erfindungsgemäßen kosmetischen Produkte einen pH-Wert zwischen 3,5 und 4,2 auf.

Die erfindungsgemäßen kosmetischen Produkte weisen 0,5-2,5 Gew.-% Phytosterole, bezogen auf das Gesamtgewicht des fertigen kosmetischen Produkts, auf.

Die Phytosterole werden erhalten aus einem Öl, ausgewählt aus der Gruppe bestehend aus Avocadoöl, Olivenöl und/oder Rapssamenöl. Die Phytosterole können in teilweise gereinigter Form eingearbeitet werden oder in Form der Öle, die reich an Phytosterolen sind, zugegeben werden.

Unter dem Begriff Phytosterole werden Steroide mit einer 3-β-Hydroxygruppe und einer Seitenkette mit 8-10 C Atomen am C17 Atom verstanden, die insbesondere aus Pflanzenöl isoliert wurden. In der Regel werden die Phytosterole als Mischungen eingesetzt, die einen überwiegenden Anteil an β-Sitosterol, einen wesentlichen Anteil an Campesterol und meist einen geringeren Anteil an Sigmasterol beinhalten.

Erfindungsgemäß ist es auch möglich den Phytosterolgehalt der eingesetzten Öle zu bestimmen und durch gezielte Auswahl der eingesetzten Öle den kosmetischen Produkten einen entsprechenden Anteil der Phytosterole zuzufügen. Eventuell noch fehlende Mengen können dadurch ergänzt werden, dass kommerziell erhältliche gereinigte Phytosterolkomplexe zugefügt werden, so dass die erfindungsgemäß bevorzugte Konzentration von 0,5-2,5 Gew.-%, noch bevorzugter 1,0-2,0 Gew.-% Phytosterol bezogen auf das fertige Produkt erreicht wird.

In einer weiteren bevorzugten Ausführungsform können die erfindungsgemäßen kosmetischen Produkte einen Gehalt an Quercetin von 10µg - 1000 µg bevorzugt 50 µg - 500 µg und besonders bevorzugt 100 µg - 250 µg pro 100g fertiges Produkt aufweisen.

Der erfindungsgemäß eingesetzte Frauenmantelextrakt beinhaltet üblicherweise 0,6-0,7 % Quercetin, bezogen auf den Trockenextrakt. Ergänzt kann dieser Gehalt werden, indem weitere Pflanzenextrakte zugegeben werden, die reich an Quercetin sind.

Quercetin ist ein zu den Flavonoiden gehörender Naturfarbstoff, der in vielen Pflanzen (Eiche, Äpfel, Weintrauben und andere) vorkommt. Bekannt ist, dass Quercetin eine antikancerogene Wirkung hat, die möglicherweise auf das antioxdative Potenzial zurückzuführen ist. Auch wenn die Wirkungsweise von Quercetin noch unbekannt ist, nimmt man an, dass es als Radikalfänger wirkt und reaktive Formen des Sauerstoffs (beispielsweise Superoxidanion) inaktiviert. Reaktive Formen des Sauerstoffs können auch in anderer Weise den Alterungsprozess der Haut beschleunigen. Daher verbessert die Zugabe von Quercetin die schützende und pflegende Wirkung der erfindungsgemäßen Produkte. Die Zugabe von Quercetin erfolgt entweder als Reinsubstanz oder bevorzugt als angereicherter Pflanzenextrakt. Bevorzugt eingesetzt werden Extrakte aus Eichenrinde, oder aus Blättern von Rosskastanie, Apfelbäumen oder Rosen.

In weiteren Ausführungsformen der vorliegenden Erfindung können auch Stoffe, wie zum Beispiel antiseptisch wirkende Stoffe, wie z.B. Chitosan eingearbeitet werden. Sinnvoll sind auch weitere antioxidativ wirkende Stoffe, wie z.B. die in Mangosteen vorkommenden Verbindungen. Derartige Stoffe werden erfindungsgemäß in einer Menge von 0,1 bis 5,0 Gew.-%, bevorzugt 0,2 bis 1,5 Gew.-% und besonders bevorzugt in einer Menge von 0,25 bis 0,5 Gew.-%, bezogen auf das fertige kosmetische Produkt, zugegeben.

Die kosmetischen Produkte liegen bevorzugt in Form einer barrierestabilisierenden Emulsion als Creme oder alternativ als Lotion vor. Der Ausdruck "barrierestabilisierende Emulsion" umfasst sowohl O/W-Lotionen und O/W-Cremen, wie auch W/O-Lotionen und W/O-Cremen.

Die erfindungsgemäßen kosmetischen Produkte können einerseits zur Verbesserung des Zustandes der Altershaut eingesetzt werden. Andererseits eignen sie sich aber ganz besonders zur Behandlung altersbedingter inflammatorischer Hautzustände.

Da es sich bei den erfindungsgemäßen kosmetischen Produkten in erster Linie um hautpflegende Produkte handelt, sind diese bevorzugterweise frei von Tensiden, die überwiegend zur Reinigung eingesetzt werden.

In einer bevorzugten Ausführungsform sind sämtliche Bestandteile der kosmetischen Produkte pflanzlichen Ursprungs. Solche Komponenten, die von Tieren, beispielsweise Talg, herstammen werden nicht verwendet. Dadurch kann eine potentielle Kontamination des Produktes mit potentiell pathogenen Agenzien wie Viren oder Prionen ausgeschlossen werden.

Die Wirkmechanismen, auf denen die überraschende synergistische Wirksamkeit der erfindungsgemäßen Kosmetikprodukte beruht, sind nicht ohne weiteres wissenschaftlich eindeutig zu erklären. Ohne an eine Theorie gebunden sein zu wollen, könnte die vorliegende Erfindung auf den nachfolgend skizzierten Grundlagen beruhen:
Die epidermale Permeabilitätsbarriere ist in der Hornschicht (Stratum corneum) lokalisiert. Sie wird aus proteinreichen Korneozyten und interzellulären Lipiddoppelschichten (Lipidmatrix) gebildet. Die interzellulären Lipide sind lamellar angeordnet und bilden so eine stark hydrophobe, nahezu impermeable Barriere, wodurch ein zu hoher Wasserverlust und das Eindringen von Fremdstoffen und Mikroorganismen verhindert werden. Folgerichtig stellt die interzelluläre Lipidmatrix die zentrale Funktionseinheit der Permeabilitätsbarriere dar.

Ein wesentlicher Aspekt der vorliegenden Erfindung ist die Bedeutung des leicht sauren physiologischen Hautoberflächen-pH (pH 4 bis 5) für die epidermale Barrierefunktion. Bildung und Formation der lamellar angeordneten Lipidmatrix ist stark pH-abhängig, was belegt werden konnte, indem der leicht saure Stratum-corneum-pH (pH 4,5 bis 5) experimentell erhöht wurde. Durch die experimentelle pH-Erhöhung wurde die Integrität und die Regeneration der epidermalen Barriere signifikant herabgesetzt.

Die lamellare Formation der Lipide wird durch die Aktivität der beiden Lipasen "β-Glucocerebrosidase" und "saure Sphingomyelinase" eingeleitet. Die für die Bildung der interzellulären Lipidmatrix erforderlichen Lipide, werden in der Epidermis im Rahmen der Differenzierung synthetisiert und als polare Lipide ("pro barrier lipids") in die Übergangszone zwischen Stratum granulosum und Stratum corneum durch Exozytose freigesetzt. Diese polaren Lipide werden dann durch die pH-abhängigen Enzyme (pH-Optimum: 4 bis 5) β-Glucocerebrosidase und saure Sphingomyelinase in apolare Barrierelipide umgewandelt und über diesen Weg in die interzelluläre Lipidmatrix des Stratum corneum eingebaut. Dieser Prozess ist durch die altersbedingte pH-Erhöhung gestört, mit der Folge einer reduzierten epidermalen Barrierefunktion im Alter. Durch die vorliegende Erfindung kann der erhöhte Altershaut-pH normalisiert und stabilisiert werden, was wiederum einen positiven Einfluss auf die Aktivität der erwähnten Lipasen hat. Die Formation der epidermalen Barriere im Alter wird dadurch günstig beeinflusst und kann als erhöhte Barriereintegrität und beschleunigte Barriereregeneration experimentell bestimmt werden.

Da der Stratum corneum an der Grenzfläche zur Umwelt angeordnet ist, ist die epidermale Barriere direkt und kontinuierlich Umweltnoxen, wie z.B. der UV-Strahlung, ausgesetzt. Es ist allgemein anerkannt, dass UV-Strahlung (insbesondere UVA) zu einer vermehrten Bildung "reaktiver Sauerstoffspezies (ROS)" in der Epidermis führt. Weiterhin wurde belegt, dass UVB- und UVA-Strahlung die Oxidation der interzellulären Lipide durch ROS im Stratum corneum induzieren und die Funktion der epidermalen Barriere herabsetzen. Neben der Oxidation wichtiger Barrierelipide und Lipidfraktionen wurde außerdem nachgewiesen, dass die an der Formation der cornealen Lipidmatrix entscheidend beteiligte β-Glucocerebrosidase UV-induziert in ihrer Aktivität gehemmt wird.

In der Altershaut sind die Mechanismen zum Schutz der epidermalen Zellen gegen oxidative Stressoren reduziert. Eine zentrale Komponente des menschlichen "antioxidativen Netzwerkes" zum Schutz von Lipiden in Biomembranen ist Vitamin E (Tocopherol). Als lipidlösliches Antioxidanz verhindert es die Lipidperoxidation mehrfach ungesättigter Fettsäuren der cornealen Lipidmatrix durch ROS. Im humanen Stratum corneum und insbesondere im Sebum konnten hohe Konzentrationen von Vitamin E nachgewiesen werden. Berücksichtigt man außerdem die reduzierte Talgdrüsenaktivität im Alter, welche zu einer Verminderung des Sebumgehaltes auf der Hautoberfläche führt, wird deutlich, dass die Vitamin E-basierte antioxidative Kapazität im Alter reduziert ist.

Durch topische Applikation von Antioxidantien wird die interzelluläre Lipidmatrix des Stratum corneum vor Lipidperoxidation und somit vor einer durch ROS bedingten Strukturveränderung geschützt. Das in der vorliegenden Erfindung eingesetzte α-Tocopherolacetat baut in der Epidermis ein "antioxidatives Schutzdepot" auf, weil es kontinuierlich durch Esterasen zum antioxidativ wirkenden α-Tocopherol biokonvertiert wird.

Durch Versuche wurde die antioxidative Kapazität des hier eingesetzten Frauenmantelextraktes mit hohem Polyphenolgehalt (Ellagtannine, Gallotannine, Flavonoide) nachgewiesen. Im Gegensatz zum α-Tocopherol, welches sich aufgrund seiner lipophilen Struktur in den Lipiddoppelschichten einlagert, entfalten Polyphenole (hohe Anzahl Hydroxylgruppen) ihre antioxidative Wirkung auch außerhalb der Lipidmatrix, also auch in hydrophilen Kompartimenten. Hinzu kommt, dass Polyphenole über einen weiteren antioxidativen Schutzmechanismus verfügen, denn sie können Metallionen, die an der Bildung von ROS beteiligt sind durch Chelatbildung binden. Somit ist, neben der "Reaktion mit Peroxylradikalen", durch die "Komplexierung von Metallionen" ein weiterer antioxidativer Mechanismus zum Schutz der epidermalen Barrierelipide gegeben.

Zudem sind die Flavonoide von besonderem Interesse, denn sie sind in der Lage die antioxidative Kapazität von α-Tocopherol, im Sinne eines synergistischen Effekts stark zu erhöhen und oxidiertes α-Tocopherol zu regenerieren.

Durch die vorliegende Erfindung wird einerseits die physiologische Bildung der interzellulären Lipidmatrix im Alter normalisiert und begünstigt (pH 3,0 bis 5,2) und andererseits über den Einsatz zweier unterschiedlich antioxidativ wirkender Komponenten (Vitamin E, Frauenmantelextrakt) geschützt. Im Rahmen der vorliegenden Erfindung werden bevorzugt Vitamin E bzw. Tocopherolacetat und Frauenmantel kombiniert eingesetzt, weil hierdurch überraschend vorteilhafte Wirkungen erzielt werden können. Darüber hinaus ist der antioxidative Schutz der epidermalen Barriere durch die unterschiedlichen chemischen Eigenschaften der kombinierten Antioxidantien (lipophil und ambiphil) auf das gesamte Stratum corneum (inklusive Hautoberfläche) ausgedehnt. Die beschriebene Wechselwirkung zwischen Flavonoiden und α-Tocopherol unterstreicht diese Aussage.

Folgerichtig wird durch die hier bevorzugte kombinierte topische Anwendung von Vitamin E und dem Frauenmantelextrakt in einer auf pH 3,2 bis 4,8 eingestellten Emulsion die epidermale Permeabilitätsbarriere der Altershaut adäquat gebildet, stabilisiert und nachhaltig geschützt. Die synergistischen Effekte der hier beschriebenen Wirkkomponenten sind die Voraussetzung für eine langfristige Funktionalität der epidermalen Permeabilitätsbarriere im Alter.

Die Zusammensetzung der einzelnen kosmetischen Produkte hängt in erster Linie davon ab, ob es sich um eine Creme, ein Öl, eine Lotion oder ein Fluid handelt. In Abhängigkeit davon kann der Wassergehalt variieren und die Zusammensetzung der einzelnen Komponenten ändert sich entsprechend. Die erfindungsgemäßen kosmetischen Produkte beinhalten in bevorzugter Ausführungsform:

| **Anteil** | **bevorzugte Bereiche** | **Komponente** |
|---|---|---|
| 0,1-5 Gew.-% | 0,15-1,5 Gew.-% | Frauenmantel |
| 0,1-4 Gew.-% | 0,2-0,8 Gew.-% | Phytosterole |
| 0,1-15 Gew.-% | 1,0-5 Gew.-% | pflanzliche Öle, insbesondere Avocadoöl, Olivenöl, Mandelöl und/oder Rapssamenöl |
| 0,01-5 Gew.-% | 1,5-2,5 Gew.-% | Tocopherolacetat |
| 0,01-2 Gew.-% | 0,1-0,8 Gew.-% | Verdicker (beispielsweise Xanthan) |
| 0,01-2 Gew.-% | 0,2-1,0 Gew.-% | Säureregulator |
| 0,01-3,0 Gew.-% | 0,05-1,0 Gew.-% | Zitronensäure (beispielsweise Zitronensäuresalze) |
| 0,01-5 Gew.-% | 0,1-1,5 Gew.-% | Emulgatoren |
| 0-10 Gew.-% | 0,5-5 Gew.-% | Alkohol |
| 40-80 Gew.-% | 50-70 Gew.-% | gereinigtes Wasser |

Die Summe der Komponenten ergibt immer 100 Gew.-%.

Die vorliegende Erfindung wird durch die nachfolgenden Beispiele weiter erläutert:

### Beispiel 1

Um den synergistischen Effekt von Emulsionen mit niedrigem pH-Wert und Frauenmantelextrakt zu belegen, wurde ein Versuch an gesunden jungen Probanden durchgeführt. Hierzu wurden zwei Emulsionen hergestellt. Die Ausgangsemulsion wies einen pH-Wert von 4,0 ± 0,1 auf und beinhaltete eine Emulsionsgrundlage, Wasser und als Vergleich 4 Gew.-% Vitamin E (α-Tocopherolum Acetat).

Die erfindungsgemäße Zusammensetzung beruhte auf derselben Salbengrundlage, wies einen pH-Wert von 4,0 ± 0,2 und als Wirkstoff 2 Gew.-% Vitamin E und 1 Gew.-% Frauenmanteltrockenextrakt auf.

Jeweils 10 Probanden applizierten eine der beiden Emulsionen 4 Wochen lang auf einen Unterarm. Anschließend wurden die Testareale UV exponiert (UVA: 4J/cm²). 48 Stunden später wurden die Testareale mittels Klebestreifenabrisse irritiert, bis der TEWL (Transepidermaler Wasserverlust) um das 3-fache angestiegen war. 24 und 48 Stunden postirritativ wurde der TEWL bestimmt (Maß der Barriereregeneration). Vergleichend ausgewertet wurden 4 Testareale:

| | Li Unterarm | | Re Unterarm | |
|---|---|---|---|---|
| | Areal 1 | Areal 2 | Areal 3 | Areal 4 |
| vorbehandelt | | | X | X |
| UVA exponiert | | X | X | |

Nachfolgend werden die Ergebnisse zusammengefasst.

Es wurde festgestellt, dass die Barriereregeneration der mit der erfindungsgemäßen Emulsion vorbehandelten Haut deutlich besser, d.h. schneller war als die mit der Vergleichsemulsion (nur Vitamin E als Wirkstoff) vorbehandelten Haut. Die erfindungsgemäße Emulsion bewirkt also 24 Stunden nach Exposition der Haut mit UVA-Licht eine bessere Barriereregeneration. Da die erfindungsgemäße Emulsion den hautphysiologischen pH-Wert gesunder junger Erwachsener nicht negativ beeinflusst, muss eine deutliche Verbesserung der Barrierefunktion gerade bei der älteren Haut angenommen werden. Bei kontinuierlicher Anwendung ist auch eine Zunahme der relativen Hornschichtfeuchte zu erwarten, so dass gerade bei der Altershaut eine deutliche Verbesserung der Barrierefunktion erwartet werden kann.

### Beispiel 2

Der in Beispiel 1 dargestellte Versuchsansatz wurde wiederholt. Es wurde eine barrierestabilisierende Emulsion hergestellt. Verglichen wurde einmal die barrierestabilisierende Emulsion, die einen pH-Wert von 4,0 ± 0,1 aufwies und keinen Wirkstoff enthielt, mit einer barrierestabilisierenden Emulsion, die im Unterschied zu der Vergleichszusammensetzung 1,0 Gew.-% eines durch wässrige Extraktion erhaltenen Trockenextrakts von Frauenmantel enthielt.

Die Versuche wurden durchgeführt wie in Beispiel 1 beschrieben. Überraschenderweise wurde festgestellt, dass die barrierestabilisierende Emulsion, die 1 Gew.-% durch wässrige Extraktion erhaltenen Trockenextrakt von Frauenmantel enthielt, eine viel bessere epidermale Barriereregeneration zeigte.

Die Ergebnisse dieses Versuchs sind in Figur 7 zusammengefasst. Die zugehörige Legende erläutert die Versuchsergebnisse.

## Patentansprüche

1. Kosmetisches Produkt mit einem pH-Wert von 3,2-4,8, **dadurch gekennzeichnet, dass** es 0,1-5 Gew.-% bezogen auf das Gewicht des Produktes eines Trockenextraktes aus Frauenmantel und 0,5-2,5 Gew.-% Phytosterole, die erhalten wurden aus einem Öl, ausgewählt aus Avocadoöl, Olivenöl und/oder Rapssamenöl, bezogen auf das Gewicht des fertigen Produktes enthält.

2. Kosmetisches Produkt nach Anspruch 1, **dadurch gekennzeichnet, dass** es einen pH-Wert zwischen 3,5 und 4,2 aufweist.

3. Kosmetisches Produkt nach Anspruch 1, **dadurch gekennzeichnet, dass** der Trockenextrakt durch eine wässrige Extraktion der Droge erhalten wurde.

4. Kosmetisches Produkt nach einem der vorhergehenden Ansprüche, **dadurch gekennzeichnet, dass** es weiterhin 0,01-5 Gew.-% bezogen auf das Gewicht des fertigen Produktes an Vitamin E oder einem Derivat hiervon enthält.

5. Kosmetisches Produkt nach Anspruch 4, **dadurch gekennzeichnet, dass** es sich bei dem Vitamin E-Derivat um Tocopherylacetat handelt.

6. Kosmetisches Produkt nach einem der vorhergehenden Ansprüche, **dadurch gekennzeichnet, dass** es einen Gehalt an Quercetin von 10 µg - 1000 µg pro 100 g fertiges Produkt aufweist.

7. Kosmetisches Produkt nach Anspruch 6, **dadurch gekennzeichnet, dass** es 2,5 bis 10 Gew.-% eines Emulgators bezogen auf das Gewicht des fertigen Produktes beinhaltet.

8. Kosmetisches Produkt nach einem der Ansprüche 1-7, **dadurch gekennzeichnet, dass** es sich um eine barrierestabilisierende Lotion handelt.

9. Kosmetisches Produkt nach einem der Ansprüche 1-7, **dadurch gekennzeichnet, dass** es sich um eine barrierestabilisierende Creme handelt.

10. Kosmetisches Produkt nach einem der Ansprüche 1-9 für die Behandlung der Altershaut.

11. Kosmetisches Produkt nach einem der Ansprüche 1-9 zur Verwendung bei der Behandlung altersbedingter inflammatorischer Hautzustände.

12. Kosmetisches Produkt nach einem der Ansprüche 1-9 zur Verwendung bei der Verbesserung der Funktion der epidermalen Permeabilitätsbarriere.

## Claims

1. A cosmetic product having a pH value of 3.2-4.8, **characterized in that** it has 0.1-5% by weight, based on the weight of the product, of a dry extract of lady's mantle and 0.5-2.5% by weight phytosterols, which were obtained from an oil selected from avocado oil, olive oil and/or rape-seed oil, based on the weight of the finished product.

2. The cosmetic product according to claim 1, **characterized in that** it has a pH value between 3.5 and 4.2.

3. The cosmetic product according to claim 1, **characterized in that** dry extract was obtained by an aqueous extraction of the drug.

4. The cosmetic product according to any one of the preceding claims, **characterized in that** it further contains 0.01-5% by weight, based on the weight of the finished product, of vitamin E or a derivative thereof.

5. The cosmetic product according to claim 4, **characterized in that** the vitamin E derivative is tocopherylacetate.

6. The cosmetic product according to any one of the preceding claims, **characterized in that** it has a content of quercetin of 10 µg - 1000 µg per 100 g of the finished product.

7. The cosmetic product according to claim 6, **characterized in that** it contains 2.5 to 10% by weight of an emulsifying agent based on the weight of the finished product.

8. The cosmetic product according to any one of claims 1-7, **characterized in that** it is a barrier-stabilizing lotion.

9. The cosmetic product according to any one of claims 1-7, **characterized in that** it is a barrier-stabilizing cream.

10. The cosmetic product according to any one of claims 1-9 for treating the aged skin.

11. The cosmetic product according to any one of claims 1-9 for use in the treatment of age-related inflammatory skin conditions.

12. The cosmetic product according to any one of claims 1-9 for use in improving the function of the epidermal permeability barrier.

## Revendications

1. Produit cosmétique avec une valeur de pH de 3,2-4,8, **caractérisé en ce qu'**il contient 0,1-5 % en poids d'un extrait sec d'alchémille par rapport au poids du produit et 0,5-2,5 % en poids de phytostérols obtenus à partir d'une huile, choisie parmi l'huile d'avocat, l'huile d'olive et/ou l'huile de colza, par rapport au poids du produit fini.

2. Produit cosmétique selon la revendication 1, **caractérisé en ce qu'**il présente une valeur de pH entre 3,5 et 4,2.

3. Produit cosmétique selon la revendication 1, **caractérisé en ce que** l'extrait sec a été obtenu par le biais d'une extraction aqueuse de la drogue.

4. Produit cosmétique selon l'une des revendications précédentes, **caractérisé en ce qu'**il contient en outre 0,01-5 % en poids de vitamine E ou d'un dérivé de celle-ci par rapport au poids du produit fini.

5. Produit cosmétique selon la revendication 4, **caractérisé en ce qu'**il s'agit de l'acétate de tocophérol dans le cas du dérivé de la vitamine E.

6. Produit cosmétique selon l'une des revendications précédentes, **caractérisé en ce qu'**il présente une teneur en quercétine de 10 *µ*g-1 000 *µ*g pour 100 g de produit fini.

7. Produit cosmétique selon la revendication 6, **caractérisé en ce qu'**il contient 2,5 à 10 % en poids d'un agent émulsifiant par rapport au poids du produit fini.

8. Produit cosmétique selon l'une des revendications 1-7, **caractérisé en ce qu'**il s'agit d'une lotion stabilisant la barrière cutanée.

9. Produit cosmétique selon l'une des revendications 1-7, **caractérisé en ce qu'**il s'agit d'une crème stabilisant la barrière cutanée.

10. Produit cosmétique selon l'une des revendications 1-9 pour le traitement du vieillissement de la peau.

11. Produit cosmétique selon l'une des revendications 1-9 pour une utilisation dans le cadre du traitement d'états cutanés inflammatoires liés au vieillissement.

12. Produit cosmétique selon l'une des revendications 1-9 pour une utilisation dans le cadre de l'amélioration de la fonction de la barrière de perméabilité épidermique.
